# EUROPÄISCHE PATENTANMELDUNG

(11) **EP 4 170 968 A1**
(43) Veröffentlichungstag der Anmeldung: **26.04.2023**
(21) Anmeldenummer: 22202524.9
(22) Anmeldetag: 19.10.2022
(51) Int. Cl.: H04L 9/40, G16H 20/17, G16H 40/67, H04L 67/12, H04L 9/32

(54) **VERFAHREN ZUM KOPPELN EINES MEDIZINISCHEN GERÄTS MIT EINEM NETZWERK**

(30) Priorität: 19.10.2021 DE 102021127120
(71) Anmelder: B. Braun Melsungen AG, 34212 Melsungen (DE)
(72) Erfinder: SCHWARZ, Jan, 34212 Melsungen (DE); ERLEN, Christoph, 34132 Kassel (DE); NIEMEIER, Carsten, 34130 Kassel (DE); BATZDORF, Stefan, 36041 Fulda (DE); KAUBA, Michael, 34277 Fuldabrück (DE); OSTERMOELLER, Michael, 36251 Bad Hersfeld (DE); HOEVEL, Stephan, 34121 Kassel (DE); RICHARDT, Mario, 34289 Zierenberg (DE)
(74) Vertreter: Winter, Brandl - Partnerschaft mbB

(57) **Zusammenfassung**

Die vorliegende Offenbarung betrifft ein Verfahren zum Aufbauen einer sicheren und vertrauenswürdigen Kommunikationsverbindung zwischen zumindest einem medizinischen Gerät (1) und einem Netzwerk (2), mit einem ersten Kommunikationskanal (3) und zumindest einem zweiten Kommunikationskanal (4) aus einer Vielzahl von zweiten Kommunikationskanälen, mit den folgenden Schritten: Senden einer Verbindungsanfrage durch das zumindest eine medizinische Gerät (1) an eine Steuerungseinheit (5), Anmelden des zumindest einen medizinischen Geräts (1) über den ersten Kommunikationskanal (3) in dem Netzwerk (2), und Verschlüsseln und Aufbauen der Kommunikationsverbindung über den zumindest einen zweiten Kommunikationskanal (4) aus der Vielzahl von zweiten Kommunikationskanälen, wobei der zumindest eine zweite Kommunikationskanal (4) je nach Datentyp und/ oder Priorisierung von Daten ausgewählt wird. Ferner betrifft die vorliegende Offenbarung ein Kommunikationssystem zum Bereitstellen einer sicheren und vertrauenswürdigen Kommunikationsverbindung zwischen zumindest einem medizinischen Gerät (1) und einem Netzwerk (2).

## Beschreibung

### Technisches Gebiet

Die vorliegende Offenbarung betrifft ein Verfahren zum Aufbauen einer sicheren und vertrauenswürdigen Kommunikationsverbindung zwischen zumindest einem medizinischen Gerät, insbesondere einer Infusionspumpe, und einem Netzwerk.

### Hintergrund der Erfindung

Die Offenbarung konzentriert sich auf eine sichere Kopplung/Verbindung von Geräten, insbesondere medizinischen Geräten, insbesondere Infusionspumpen mit einer klinischen IT-Plattform/ einem klinischen Server/ einem Netzwerk. Ein Aufbau einer vertrauenswürdigen Verbindung/ Kommunikationsverbindung dient zum Nachweis der Authentizität, Vertraulichkeit und Integrität zwischen zumindest einem medizinischen Geräten und einer klinischen IT-Plattform/ einem klinischen Netzwerk innerhalb eines nicht vertrauenswürdigen Krankenhausnetzwerks.

### Stand der Technik

In den derzeitigen modernen Implementierungen basiert das Koppeln/ Verbinden in der Regel zunächst auf dem manuellen Austausch von Sicherheitsnachweisen. Bekannte Standards basieren hierbei beispielsweise auf Nachweisen wie Simple Certificate Enrollment Protocol (SCEP) oder Enrollment over Secure Transport (EST).

Aktuelle Lösungen setzen eine vorkonfigurierte Authentizität zwischen dem zumindest einen medizinischen Gerät und dem Server/ Netzwerk voraus, beispielsweise durch bereits definierte vertrauenswürdige Zertifikate. Diese Zertifikate werden runtergeladen und somit der Datenverkehr verschlüsselt.

Vor dem Hintergrund liegt der vorliegenden Offenbarung die Aufgabe zugrunde, die Nachteile aus dem Stand der Technik zu beseitigen oder zumindest zu vermindern. Demnach ist es ein Ziel der vorliegenden Offenbarung die folgenden Probleme lösen:
- Nachweis der Beziehung zwischen der digitalen und der physischen Identität des zumindest einen medizinischen Geräts;
- Ermöglichung der Verschlüsselung für (asynchrone) Nachrichten mit unbekannten Empfängern;
- Verifizierung der Integrität und Authentizität von Nachrichten;
- Aussperrung unbekannter/unvertrauter Geräte; und
- Sofortiger Entzug der Vertrauensbeziehung zu jeder Zeit.

Die Aufgabe wird gelöst durch ein Verfahren zum Aufbauen einer sicheren und vertrauenswürdigen Kommunikationsverbindung zwischen zumindest einem medizinischen Gerät, insbesondere zumindest einer Infusionspumpe, und einem Netzwerk gemäß den Merkmalen des Anspruchs 1. Nachfolgend ist unter einem "Client" zumindest ein medizinisches Gerät zu verstehen.

Die sichere und vertrauenswürdige Kommunikationsverbindung zwischen zumindest einem medizinischen Gerät und einem Netzwerk wird mit einem ersten Kommunikationskanal und zumindest einem zweiten Kommunikationskanal aus einer Vielzahl von zweiten Kommunikationskanälen, mit den nachfolgenden Schritten aufgebaut. In einem ersten Schritt wird eine Verbindungs-/ Kopplungsanfrage durch das zumindest eine medizinische Gerät an eine Steuerungseinheit gesendet. In einem darauffolgenden Schritt erfolgt das Anmelden des zumindest einen medizinischen Geräts über den ersten Kommunikationskanal in dem Netzwerk. In einem letzten Schritt wird die Kommunikationsverbindung über den zumindest einen zweiten Kommunikationskanal aus der Vielzahl von zweiten Kommunikationskanälen verschlüsselt und aufgebaut, wobei der zumindest eine zweite Kommunikationskanal je nach Datentyp und/ oder Priorisierung von Daten ausgewählt wird.

In anderen Worten wird im Rahmen der Installation/ Verbindung/ Kopplung des zumindest einen medizinischen Geräts und des IT-Systems/ des Netzwerks ein bekanntes "Zugangsgeheimnis" ausgetauscht. An dem zumindest einen medizinischen Gerät beginnt der Bediener/ Anwender die Kopplungs-/ Pairing Sequenz, indem er die Kopplung/ Verbindung über einen vorzugsweise gesicherten, ersten Kommunikationskanal anfordert. Nach einer optionalen Bestätigung der Pairing-Anforderung durch den IT-Systembetreiber richtet das System dedizierte zweite Kommunikationskanäle für das zumindest eine medizinische Gerät ein. Das System generiert Zertifikate und verteilt das Zertifikat über die dedizierten Kommunikationskanäle. Am Ende des Kopplungs-/ Pairing-Prozesses sind die Kommunikationskanäle zwischen Gerät und IT-System durch die generierten Sicherheitszertifikate geschützt.

Ein solches Kopplungsverfahren hat den Vorteil, dass bereits für den Anmeldungsschritt mehrere medizinische Geräte den gleichen ersten Kommunikationskanal verwenden können und auch in Bezug auf die zweiten Kommunikationskanäle eine Mehrfachnutzung von verschiedenen medizinischen Geräten möglich sind. Auf diese Weise können eine Menge Kommunikationskanäle eingespart werden.

In anderen Worten ausgedrückt kann der Verbindungs-/ Kopplungsprozess für ein neues medizinisches Gerät durch folgende Abschnitte beschrieben werden. Wenn ein neues medizinisches Gerät dem Netzwerk beitritt, muss das zumindest eine medizinische Gerät eine Pairing-/ Verbindungs-/ Kopplungsanfrage an die Steuerungseinheit senden. Sobald eine Anwendung der Steuerungseinheit eine Anfrage erhält, hat der Bediener/ Anwender die Möglichkeit, die Verbindungsanfrage zu akzeptieren oder abzulehnen, damit das zumindest eine medizinische Gerät dem Netzwerk beitreten kann.

Bevor der Verbindungs-/ Kopplungsprozess eingeleitet wird, muss das zumindest eine medizinische Gerät für die Verbindung mit dem Netzwerk vor Ort konfiguriert werden. Darüber hinaus muss das zumindest eine medizinische Gerät mit der IT-Adresse eines Message Brokers konfiguriert werden. Unter dem Message Broker ist ein Kommunikationssystem zu verstehen, das auf mehreren, verteilten Servern laufen kann, die einen Datenverkehr unter sich aufteilen und einen ausfallsicheren Betrieb unterstützen. In diesem Kommunikationssystem werden Informationen mit einem Zeitstempel versehen und in sogenannten Topics gespeichert. Die gespeicherten Informationen werden im Message Broker repliziert und verteilt und stehen Server Anwendungen zur weiteren Verarbeitung zur Verfügung.

Weitere Ausführungsformen sind nachfolgend gemäß den Aspekten der Unteransprüche beschrieben.

Es ist bevorzugt, wenn der erste Kommunikationskanal ein Registrierungskanal ist und die Anmeldung ein einmaliger Verfahrensschritt je medizinischem Gerät ist, wobei in dem Verfahrensschritt der Anmeldung das zumindest eine medizinische Gerät mittels eines Verifikationsschritts für die Kommunikationsverbindung konfiguriert wird. Hierbei läuft die Steuerungseinheit auf einem Server, der mit dem Netzwerk verbunden ist.

Es ist von Vorteil, wenn in dem Verifikationsschritt die Steuerungseinheit einen Verbindungsmodus vorgibt, welcher insbesondere eine optische Verifikation, eine manuelle Verifikation oder eine einfache Verbindung ohne Verifikation beinhaltet.

In anderen Worten gibt es drei verschiedene Modi für das Pairing/ Koppeln/ Verbinden zumindest eines medizinischen Geräts mit dem klinischen Netzwerk. Ein erster Modus ist die optische Verifizierung. Hierbei wird zusätzlich ein mobiles Endgerät benötigt, um einen QR-Code zu scannen und darin enthaltene Informationen auszulesen. Ein zweiter Modus ist die manuelle Verifizierung und ein dritter Modus ist ein einfaches Koppeln/ Verbinden, bei welchem keine Verifizierung notwendig ist und eine reduzierte Vertrauensstufe hat. Der dritte Modus ist nur in einer gesicherten Netzwerkumgebung zu verwenden. Bei der manuellen Verifizierung zeigt das zumindest eine medizinische Gerät einen Schlüsselbestätigungscode kodiert in einem Display an und der Bediener/ Anwender kann diesen an der Steuerungseinheit bestätigen, woraufhin die Steuerungseinheit weitere Befehle sendet.

Die Auswahl, welche der drei Verifizierungsmodi verwendet wird, wird vollständig von einer Anwendung der Steuerungseinheit gesteuert. Eine Anwendung auf Seite des zumindest einen medizinischen Geräts würde immer das einfache Koppeln/ Verbinden gemäß dem dritten Modus verwenden, da dies in einer vertrauenswürdigen Netzwerkumgebung ausgeführt wird. Das heißt, das einfache Koppeln/ Verbinden ist eine erste Ausbaustufe, welche durch die optische bzw. manuelle Verifizierung in eine Zwei-Faktor Authentifizierung ausgebaut ist.

Für das Verbinden/ Koppeln des zumindest einen medizinischen Geräts gibt es folgende Voraussetzungen:
- das zumindest eine medizinische Gerät muss für die Verbindung mit dem Netzwerk konfiguriert sein,
- das zumindest eine medizinische Gerät muss die Adresse des Message Brokers kennen, wobei vorzugsweise die Verwendung eines vordefinierten dns-Eintrags als Fallback zu verwenden ist,
- das zumindest eine medizinische Gerät muss das Zugangsgeheimnis/ den Schlüssel (Hospital Access Secret) kennen
- Optional kann das zumindest eine medizinische Gerät ein vertrauenswürdiges Stammzertifikat (CertCA) haben.

Es ist bevorzugt, wenn im Verfahrensschritt der Verschlüsselung eine mehrfache Verwendung eines Schlüssels für mehrere medizinische Geräte vorgesehen ist.

Es ist von Vorteil, wenn die Vielzahl zweiter Kommunikationskanäle dazu vorgesehen und ausgebildet sind, um jeweils unterschiedliche Schlüssel bereitzustellen, wobei die unterschiedlichen Schlüssel je nach Datentyp und/ oder Priorisierung der Daten unterschiedliche Kriterien, insbesondere Sicherheitskriterien, erfüllen.

Es ist vorteilhaft, wenn in dem Verschlüsselungsschritt die Steuerungseinheit den Aufbau der Kommunikationsverbindung bestätigt, wobei die Kommunikationsverbindung mit einem Netzwerkschlüssel und einer Netzwerkschlüssel-ID verschlüsselt wird.

Es ist bevorzugt, wenn das zumindest eine medizinische Gerät den Netzwerkschlüssel und die Netzwerkschlüssel-ID empfängt und in einem persistenten Speicher speichert.

Es ist von Vorteil, wenn dem zumindest einen medizinischen Gerät der Schlüssel des zumindest einen zweiten Kommunikationskanals bekannt ist.

In anderen Worten lässt sich das Verfahren auch in die folgenden fünf Schritte unterteilen:
In einem ersten Schritt geht das zumindest eine medizinische Gerät in einen Verbindungs-/ Kopplungsmodus. Dies kann in Abhängigkeit von bestimmten Kriterien automatisch geschehen oder manuell von einer Benutzeroberfläche des zumindest einen medizinischen Gerät initiiert werden. Das zumindest eine medizinische Gerät erzeugt hierbei sein eigenes statisches EC-Schlüsselpaar und speichert dieses in dem persistenten lokalen Speicher. Ein statisches EC-Schlüsselpaar (ein Elliptic Curve Schlüssel) wird initial von dem zumindest einen medizinischen Gerät generiert. Die Steuerungseinheit leitet daraus ein vergängliches (ephemerales) Schlüsselpaar ab.

Das zumindest eine medizinische Gerät baut eine TLS-Verbindung zu dem Broker/ Vermittler unter Verwendung eines Zertifikats auf. Sofern kein vertrauenswürdiges Stammzertifikat verfügbar ist, muss das zumindest eine medizinische Gerät die TLS-Zertifikatsvalidierung deaktivieren. TLS (Transport Layer Security) ist auch bekannt unter der Vorgängerbezeichnung Secure Sockets Layer (SSL) und ist ein Verschlüsselungsprotokoll zur sicheren Datenübertragung im Internet. Das zumindest eine medizinische Gerät sendet eine Verbindungs-/Kopplungs-/Pairing-Anfrage an ein erstes Topic "ais.register.<client>" und verwendet das folgende Zugangsgeheimnis/ den Schlüssel zur Verschlüsselung:
- eine Nutzlast (zu übertragende Daten) enthält eine Zertifikatssignierungsanforderung für das EC-Schlüsselpaar des zumindest einen medizinischen Geräts;
- die Nutzlast enthält Informationen des zumindest einen medizinischen Geräts zur Anzeige an der Steuerungseinheit;
- Kennung, Gerätetyp, Familie, Seriennummer, Firmware-Version, usw.; und
- das Nutzlast-Verbindungs-Statusfeld gibt eine Verbindungsanforderung aus.

In einem zweiten Schritt versucht das zumindest eine medizinische Gerät vom Message Broker ein zweites Topic "ais.<client>.<client_identifier>" zu abonnieren (das heißt von diesem Topic Nachrichten zu empfangen), dessen Bereitstellung im Message Broker von der Steuerungseinheit wie nachfolgend beschrieben initiiert wird.

Die Steuerungseinheit konsumiert Nachrichten aus dem ersten Topic und zeigt eine Liste der medizinischen Geräte an, die eine Verbindung/ Paarung/ Kopplung angefordert haben. Der Bediener/ Anwender hat hierbei die Wahl, die Anfrage zu akzeptieren oder abzulehnen. Falls der Bediener die Anforderung/ Anfrage annimmt, erzeugt die Steuerungseinheit ein temporäres ephemeres EC-Schlüsselpaar. Die Steuerungseinheit erstellt das zweite Topic "ais.<client>.<client_identifier>" und setzt die entsprechenden Zugriffssteuerungsebenen für die Identität der Zertifikate fest. Die Steuerungseinheit leitet die Zertifikatssignierungsanforderung für das EC-Schlüsselpaar des zumindest einen medizinischen Geräts an die interne Infrastruktur weiter und erhält das signierte Zertifikat. Die Steuerungseinheit fügt die entsprechenden Zugriffssteuerungsebenen für die Identität von dem entsprechenden Zertifikat zu einem dritten Topic "ais.command.<client>" hinzu. Die Steuerungseinheit erzeugt eine Verbindungs-/ Kopplungsbefehl bzw. eine Antwort an das medizinische Gerät auf das zweite Topic unter Verwendung von dem Zugangsgeheimnis/ den Schlüsseln zur Verschlüsselung. Hierbei enthält die Nutzlast den temporären, ephemeren öffentlichen Schlüssel der Steuerungseinheit, das signierte Zertifikat des medizinischen Geräts und das Sicherheitszertifikat. Darüber hinaus enthält das Verbindungs-/ Kopplungs-/ Pairing-Statusfeld der Nutzlast die Optionen für eine optische Verifikation, eine manuelle Verifikation oder eine einfache Verifikation.

In einem dritten Schritt konsumiert das zumindest eine medizinische Gerät den Befehl zur Verifikation aus dem zweiten Topic. Das zumindest eine medizinische Gerät und die Steuerungseinheit können nun ein gemeinsames Geheimnis "z" zur Schlüsselerstellung berechnen. Das zumindest eine medizinische Gerät speichert das empfangene, signierte Zertifikat und verwendet es zur Erneuerung der TLS-Verbindung. Das zumindest eine medizinische Gerät erzeugt eine Schlüsselbestätigung. Je nach Pairing-/ Kopplungs-/ Verbindungsmodus wird diese Schlüsselbestätigung mit dem "PAIRING-VERIFIED"-Befehl an das dritte Topic zur Verschlüsselung übertragen:
a. Beim einfachen Koppeln/Verbinden sendet das zumindest eine medizinische Gerät das Kommando/ den Befehl (PAIRING_SIMPLE) der Verifikationsart selbst.
b. Beim manuellen Koppeln/Verbinden zeigt das zumindest eine medizinische Gerät den Schlüssel-Bestätigungscode kodiert im Display an und der Bediener/ Anwender kann an der Steuerungseinheit bestätigen, dass die Bestätigung korrekt ist. Daraufhin sendet die Steuerungseinheit den Befehl (PAIRING_CODE).
c. Beim optischen Koppeln/ Verbinden zeigt das zumindest eine medizinische Gerät den Schlüsselbestätigungscode als QR-Code im Display an und der Bediener/ Anwender kann ihn mit einem mobilen Endgerät über einen zweiten gesicherten Kommunikationskanal an die Steuerungseinheit senden, wobei die Steuerungseinheit den Befehl sendet.

Die Steuerungseinheit prüft, ob der Schlüsselbestätigungscode identisch ist.

In einem vierten Schritt ändert die Steuerungseinheit die Zugriffssteuerungsebenen für das zweite Topic und entfernt die Identität des entsprechenden Zertifikats. Die Steuerungseinheit sendet ein Kommando/ Befehl PAIRING_CONFIRMED an das zweite Topic und verwendet das Geheimnis "z" zur Verschlüsselung. Die Nutzlast enthält den Netzwerkschlüssel und die Netzwerkschlüssel-ID für die weitere Verschlüsselung. Das zumindest eine medizinische Gerät empfängt den Befehl/ das Kommando PAIRING_CONFIRMED und speichert den empfangenen Netzwerkschlüssel und die Netzwerkschlüssel-ID in dem persistenten Speicher.

In einem fünften Schritt sendet das zumindest eine medizinische Gerät ein PAIRING_FINISHED-Kommando/-Befehl an das dritte Topic und verwendet den Netzwerkschlüssel zur Verschlüsselung. Das zumindest eine medizinische Gerät zeigt "Verbindung erfolgreich" an. Die Steuerungseinheit fügt die entsprechenden Zugriffssteuerungsebenen für die Identität von dem Sicherheitszertifikat zu den anderen gemeinsamen Themen hinzu. Die Steuerungseinheit veröffentlicht eine Nachricht an das Thema des vierten Topics "ais.meta.<client>" mit dem Sicherheitszertifikat. Das zumindest eine medizinische Gerät verbindet sich mit den entsprechenden Topics und beginnt Daten für das Netzwerk zu produzieren.

Ferner betrifft die vorliegende Offenbarung ein Kommunikationssystem zum Bereitstellen einer sicheren und vertrauenswürdigen Kommunikationsverbindung zwischen zumindest einem medizinischen Gerät und einem Netzwerk, mit dem zumindest einen medizinischen Gerät, insbesondere einer Infusionspumpe, dem Netzwerk, einer Steuerungseinheit, die dazu vorgesehen ist, um eine Kopplungsanfrage des zumindest einen medizinischen Geräts zu empfangen, einem ersten Kommunikationskanal, der dazu ausgebildet und vorgesehen ist, um das zumindest eine medizinische Gerät in dem Netzwerk anzumelden, und zumindest einem zweiten Kommunikationskanal aus einer Vielzahl zweiter Kommunikationskanäle, der dazu ausgebildet und vorgesehen ist, die Kommunikationsverbindung zu verschlüsseln, wobei zumindest einer der Vielzahl zweiter Kommunikationskanäle je nach Datentyp und/ oder Priorisierung von Daten auswählbar ist.

Es ist bevorzugt, wenn das Kommunikationssystem dazu vorgesehen und ausgebildet ist, das Verfahren nach einem der vorstehenden Aspekte auszuführen und/oder durchzuführen.

Kurzbeschreibung der Figuren
Fig. 1 ist eine Darstellung zur Veranschaulichung eines Verbindungs-/Kopplungsaufbaus gemäß der vorliegenden Offenbarung; und
Fig. 2 ist eine Darstellung zur Veranschaulichung eines Kommunikationssystems gemäß der vorliegenden Offenbarung.

### Beschreibung der Ausführungsbeispiele

Nachstehend werden Ausführungsbeispiele der vorliegenden Offenbarung auf der Basis der zugehörigen Figuren beschrieben.

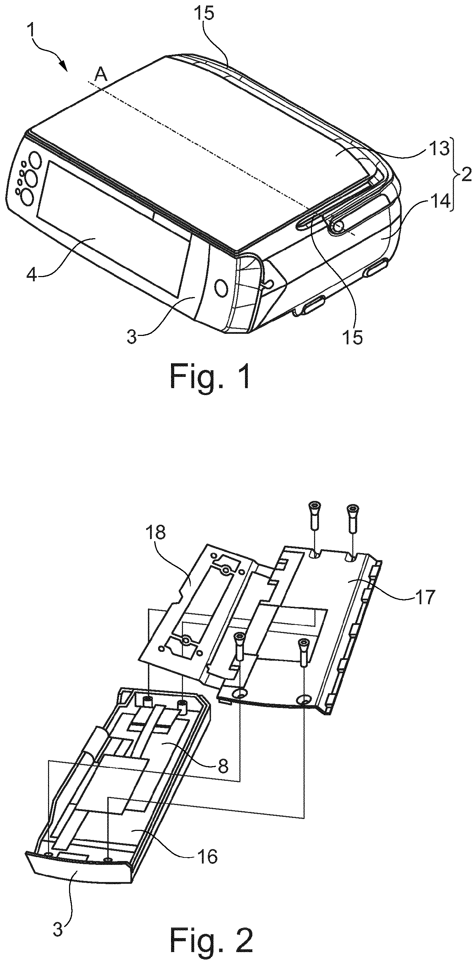

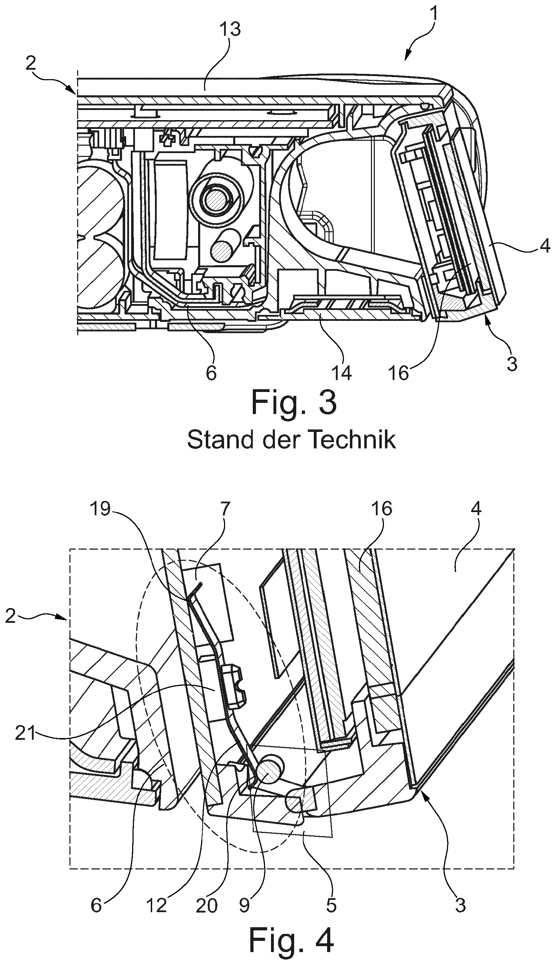

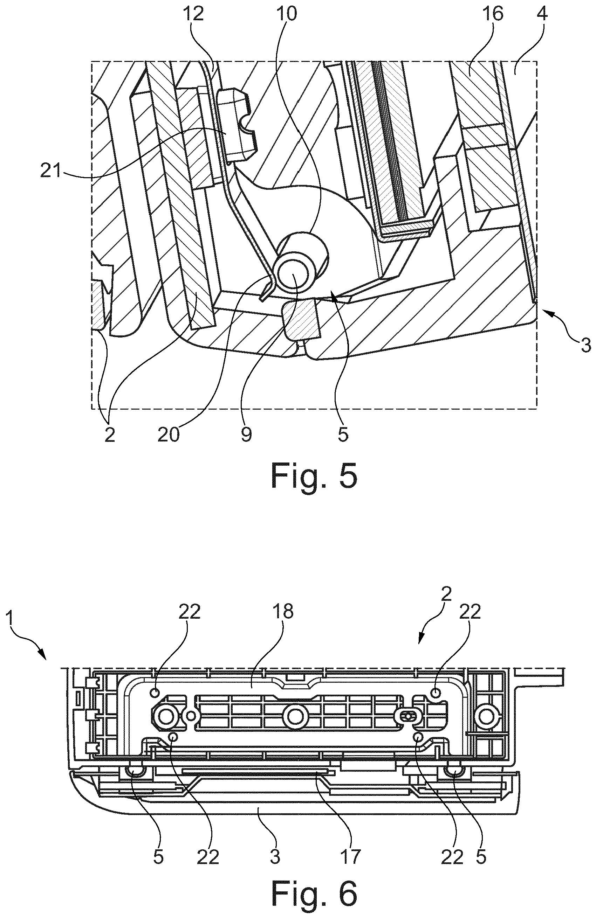

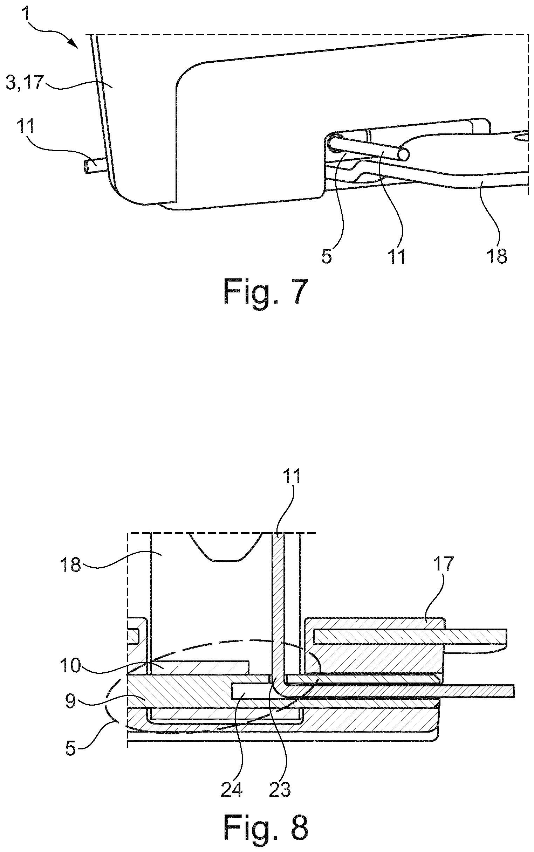

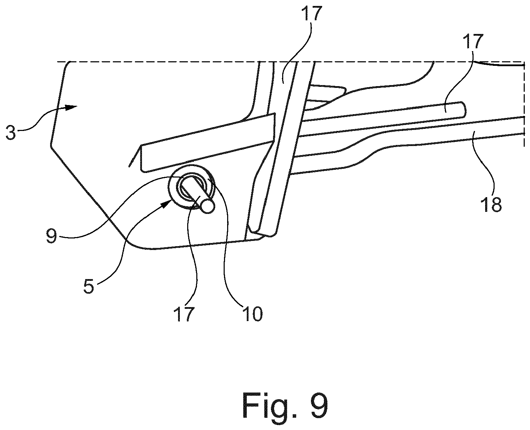

Fig. 1 ist eine Darstellung zur Veranschaulichung eines sicheren und vertrauenswürdigen Verbindungs-/Kopplungsaufbaus gemäß der vorliegenden Offenbarung. In einem ersten Schritt S1 wird von zumindest einem medizinischen Instrument 1 über einen Anwender 6 oder automatisch eine Verbindung/ Kopplung initiiert. Hierbei wird eine Verbindungsanfrage durch das zumindest eine medizinische Gerät 1 an eine Steuerungseinheit 5 gemäß einem zweiten Schritt S2 gesendet. Insbesondere wird eine Verbindungsanfrage gemäß S2 an ein Topic T1 "ais.register.<client>" gesendet und ein Zugangsgeheimnis/ ein Schlüssel zur Verschlüsselung verwendet. In einem weiteren Schritt S3 wird die Verbindungsanfrage verarbeitet. Hierbei verarbeitet die Steuerungseinheit 5 Nachrichten aus dem Topic T1 und zeigt eine Liste der medizinischen Geräte 1 an, die eine Paarung/ Verbindung/ Kopplung gemäß S2 angefordert haben. Hierbei kann der Anwender 6 entscheiden, die Verbindungsanfrage anzunehmen oder abzulehnen.

In einem Schritt S4 erstellt die Steuerungseinheit ein Topic T2 "ais.<client>.<client_identifier>", wobei das zumindest eine medizinische Gerät 1 versucht periodisch das Topic 2 zu abonnieren, bis dieses erstellt ist. Die Steuerungseinheit 5 erzeugt ein temporäres ephemeres EC-Schlüsselpaar und, sofern der Anwender 6 die Verbindungsanfrage angenommen hat, setzt die Steuerungseinheit 5 an Topic T2 die entsprechenden Zugriffssteuerungsebenen für die Identität von entsprechenden Zertifikaten. Währenddessen zeigt ein Display des zumindest einen medizinischen Geräts an, dass die Verbindung/ Kopplung in Arbeit ist.

In einem fünften Schritt S5 erzeugt die Steuerungseinheit 5, nachdem die Verbindung/ Kopplung genehmigt/ anerkannt ist, einen Kopplungsbefehl an das zumindest eine medizinische Gerät 1 des Topics T2 unter Verwendung eines Schlüssels/ Zugangsgeheimnisses zur Verschlüsselung. Das zumindest eine medizinische Gerät 1 ist nun in einem Kopplungs-/Verbindungsmodus und verarbeitet einen Befehl aus dem Topic T2.Der Befehl aus dem Topic 2 zeigt die Art der Verifikation auf. Hierbei kann zwischen einem der drei Befehle gewählt werden: "pairing_qrcode", "pairing_code" oder "pairing:simple". Das zumindest eine medizinische Gerät 1 und die Steuerungseinheit 5 können jetzt ein gemeinsames Geheimnis "z" berechnen.

Die Steuerungseinheit 5 leitet zumindest eine Zertifikatssignierungsanforderung für das EC-Schlüsselpaar des zumindest einen medizinischen Geräts 1 an eine interne Zertifizierungsinfrastruktur weiter und erhält ein signiertes Zertifikat. In einem Schritt S6 fügt die Steuerungseinheit 5 entsprechende Zugriffssteuerungsebenen für die Identität der Zertifikate zu einem Topic T3 "ais.command.<client>" hinzu. Je nach Verbindungs-/ Kopplungsmodus wird eine Schlüsselbestätigung mit dem Befehl "Verbindung verifiziert" an das Topic T3 mit dem Geheimnis "z" zur Verschlüsselung gemäß einem Schritt S7 übertragen.

Sofern die Schlüsselbestätigung durch die Steuerungseinheit 5 geprüft wurde, ändert die Steuerungseinheit 5 gemäß Schritt S8 die Zugriffssteuerungsebenen für das Topic 2 und entfernt die entsprechenden Identitäten des entsprechenden Zertifikats. In einem Schritt S9 sendet die Steuerungseinheit 5 einen Befehl "Verbindung bestätigt" an das Topic T2 und verwendet das Geheimnis "z" zur Verschlüsselung. Daraufhin weist eine Nutzlast, das heißt die Kommunikationsdaten zwischen dem zumindest einen medizinischen Gerät 1 und der Steuerungseinheit 5, einen Plattformschlüssel und eine Plattformschlüssel-ID für die weitere Verschlüsselung auf. Das zumindest eine medizinische Gerät 1 empfängt den Befehl "Verbindung bestätigt" und speichert den empfangenen Plattformschlüssel und die Plattformschlüssel-ID in einem persistenten/ nichtflüchtigen Speicher.

In einem vorletzten Schritt S10 sendet das zumindest eine medizinische Gerät 1 einen Befehl "Verbindung abgeschlossen" an das Topic T3 und verwendet den Plattformschlüssel zur Verschlüsselung. Hierbei zeigt das zumindest eine medizinische Gerät 1 an "Verbindung erfolgreich". Die Steuerungseinheit 5 fügt entsprechende Zugriffssteuerungsebenen für die Identität des Zertifikats zu einem vierten Topic T4 "ais.meta.<client>" hinzu und die Steuerungseinheit 5 veröffentlicht eine Nachricht an den Topic 4 mit dem entsprechenden Zertifikat. Daraufhin ist die Kommunikationsverbindung zwischen dem zumindest einen medizinischen Gerät 1 und dem Netzwerk 2 aufgebaut.

Fig. 2 ist eine Darstellung zur Veranschaulichung eines Kommunikationssystems 8 gemäß der vorliegenden Offenbarung. Hierbei sind beispielhaft vier medizinische Geräte 1 gezeigt. Jedes der gezeigten medizinischen Geräte 1 kann über einen ersten Kommunikationskanal 3 den Anmeldungsschritt gemäß S4 bis S7 durchführen. Des Weiteren sind in Fig. 2 beispielhaft drei zweite Kommunikationskanäle 4 angezeigt. Jedes der gezeigten medizinischen Geräte 1 ist dazu ausgebildet und vorgesehen um über zumindest einen der dargestellten zweiten Kommunikationskanäle 4 einen verschlüsselten Kommunikationskanal zu einem Netzwerk 2 aufzubauen. Die Wahl des zweiten Kommunikationskanals 4 wird in Abhängigkeit der zu übertragenden Daten/ Informationen von einer Steuerungseinheit 5 getroffen.

### Bezugszeichen

- 1: medizinisches Gerät
- 2: Netzwerk
- 3: erster Kommunikationskanal
- 4: zweiter Kommunikationskanal
- 5: Steuerungseinheit
- 6: Anwender
- 7: Verbindungsmodus
- 8: Kommunikationssystem
- T1: ais.register.<client>
- T2: ais.<client>.>client_identifier>
- T3: ais.command.<client>
- T4: ais.meta.<client>

## Patentansprüche

1. Verfahren zum Aufbauen einer sicheren und vertrauenswürdigen Kommunikationsverbindung zwischen zumindest einem medizinischen Gerät (1), insbesondere zumindest einer Infusionspumpe, und einem Netzwerk (2), mit einem ersten Kommunikationskanal (3) und zumindest einem zweiten Kommunikationskanal (4) aus einer Vielzahl von zweiten Kommunikationskanälen, mit den folgenden Schritten:
- Senden einer Verbindungsanfrage (S2 und S3) durch das zumindest eine medizinische Gerät (1) an eine Steuerungseinheit (5),
- Anmelden des zumindest einen medizinischen Geräts (1) über den ersten Kommunikationskanal (3) in dem Netzwerk (2) (S4 bis S7), und
- Verschlüsseln und Aufbauen der Kommunikationsverbindung über den zumindest einen zweiten Kommunikationskanal (4) aus der Vielzahl von zweiten Kommunikationskanälen (S8 bis S11), wobei der zumindest eine zweite Kommunikationskanal (4) je nach Datentyp und/ oder Priorisierung von Daten ausgewählt wird.

2. Verfahren nach Anspruch 1, **dadurch gekennzeichnet, dass** der erste Kommunikationskanal (3) ein Registrierungskanal ist und die Anmeldung ein einmaliger Verfahrensschritt je medizinischem Gerät (1) ist, wobei in dem Verfahrensschritt der Anmeldung das zumindest eine medizinische Gerät (1) mittels eines Verifikationsschritts für die Kommunikationsverbindung konfiguriert wird.

3. Verfahren nach Anspruch 1 oder 2, **dadurch gekennzeichnet, dass** in dem Verifikationsschritt die Steuerungseinheit (5) einen Verbindungsmodus (6) vorgibt, welcher insbesondere eine optische Verifikation, eine manuelle Verifikation oder keine Verifikation beinhaltet.

4. Verfahren nach einem vorhergehenden Anspruch 1 bis 3, **dadurch gekennzeichnet, dass** im Verfahrensschritt der Verschlüsselung eine mehrfache Verwendung eines Schlüssels für mehrere medizinische Geräte vorgesehen ist.

5. Verfahren nach einem vorhergehenden Anspruch 1 bis 4, **dadurch gekennzeichnet, dass** die Vielzahl zweiter Kommunikationskanäle (4) dazu vorgesehen und ausgebildet sind, um jeweils unterschiedliche Schlüssel bereitzustellen, wobei die unterschiedlichen Schlüssel je nach Datentyp und/ oder Priorisierung der Daten unterschiedliche Kriterien, insbesondere Sicherheitskriterien, erfüllen.

6. Verfahren nach einem vorhergehenden Anspruch 1 bis 5, **dadurch gekennzeichnet, dass** in dem Verschlüsselungsschritt die Steuerungseinheit (5) den Aufbau der Kommunikationsverbindung bestätigt, wobei die Kommunikationsverbindung mit einem Netzwerkschlüssel und einer Netzwerkschlüssel-ID verschlüsselt wird.

7. Verfahren nach Anspruch 6, **dadurch gekennzeichnet, dass** das zumindest eine medizinische Gerät (1) den Netzwerkschlüssel und die Netzwerkschlüssel-ID empfängt und in einem persistenten Speicher speichert.

8. Verfahren nach einem vorhergehenden Anspruch 1 bis 7, **dadurch gekennzeichnet, dass** dem zumindest einen medizinischen Gerät (1) der Schlüssel des zumindest einen zweiten Kommunikationskanals (4) bekannt ist.

9. Kommunikationssystem (8) zum Bereitstellen einer sicheren und vertrauenswürdigen Kommunikationsverbindung zwischen zumindest einem medizinischen Gerät (1) und einem Netzwerk (2), mit:
dem zumindest einen medizinischen Gerät (1), insbesondere einer Infusionspumpe,
dem Netzwerk (2),
einer Steuerungseinheit (5), die dazu vorgesehen ist, um eine Verbindungsanfrage des zumindest einen medizinischen Geräts (1) zu empfangen,
einem ersten Kommunikationskanal (3), der dazu ausgebildet und vorgesehen ist, um das zumindest eine medizinische Gerät (1) in dem Netzwerk (2) anzumelden, und
zumindest einem zweiten Kommunikationskanal (4) aus einer Vielzahl zweiter Kommunikationskanäle, der dazu ausgebildet und vorgesehen ist, die Kommunikationsverbindung zu verschlüsseln, **dadurch gekennzeichnet, dass**
zumindest einer der Vielzahl zweiter Kommunikationskanäle je nach Datentyp und/ oder Priorisierung von Daten auswählbar ist.

10. Kommunikationssystem nach Anspruch 9, das dazu vorgesehen und ausgebildet ist, das Verfahren nach einem der Ansprüche 1 bis 8 auszuführen und/oder durchzuführen.
